Europäisches Patentamt

European Patent Office

Office européen des brevets

(11) Numéro de publication: **0 026 832**
**B1**

(19)

(12) **FASCICULE DE BREVET EUROPEEN**

(45) Date de publication du fascicule du brevet: **17.11.82**

(51) Int. Cl.³: **C 07 C 99/12**

(21) Numéro de dépôt: **80105030.3**

(22) Date de dépôt: **25.08.80**

(54) **Procédé de purification de l'isoleucine.**

(30) Priorité: **04.10.79 CH 8949/79**

(43) Date de publication de la demande:
**15.04.81 Bulletin 81/15**

(45) Mention de la délivrance du brevet:
**17.11.82 Bulletin 82/46**

(84) Etats contractants désignés:
**BE DE FR GB NL**

(56) Documents cités:
**CHEMICAL ABSTRACTS, vol. 72, no. 15, 13 avril 1970, réf. 79483c, page 454 Columbus, Ohio, U.S. N. SAO et al.: "Isoleucine"**

**CHEMICAL ABSTRACTS, vol. 48, no. 19, 10 octobre 1954, réf. 11737b, page 1954 Columbus, Ohio, U.S. Y. ZSUCHIYA: "Separation of leucine-isoleucine mixture"**

**CHEMICAL ABSTRACTS, vol. 86, no. 23, 6 juin 1977, réf. 167493a, page 240 Columbus, Ohio, U.S. I. CHIBATA et al.: "Leucine purification"**

(73) Titulaire: **SOCIETE DES PRODUITS NESTLE S.A.**
**Case postale 353**
**CH-1800 Vevey (CH)**
㊾ **BE FR GB NL**
(73) Titulaire: **MAGGI A.G.**
**CH-8310 Kempttal (CH)**
㊾ **DE**

(72) Inventeur: **Bertholet, Raymond**
**Avenue des Alpes 66**
**CH-1814 La Tour-de-Peilz (CH)**
Inventeur: **Hirsbrunner, Pierre**
**Route de la Crottaz 30**
**CH-1802 Corseaux (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Procédé de purification de l'isoleucine

La présente invention a pour objet un procédé de purification de l'isoleucine à partir d'un mélange de leucine et d'isoleucine. Outre le procédé classique de séparation de la leucine et de l'isoleucine qui repose sur la différence de solubilité de leurs complexes ou sels de cuivre dans le méthanol et dont le rendement est très faible, on connaît par le brevet d'invention allemand No. 24 17 375 un procédé d'enrichissement de l'isoleucine industriellement réalisable basé sur une cristallisation fractionée des sels de cuivre de la leucine et de l'isoleucine à différents pH dans le domaine acide. Le degré de pureté de l'isoleucine obtenue par ce dernier procédé ne dépasse cependant pas 80%.

Le présent procédé doit permettre de mieux séparer encore l'isoleucine de la leucine et d'atteindre un haut degré de pureté tout en conservant un rendement industriellement intéressant.

Le présent procédé est caractérisé par le fait que l'on précipite l'isoleucine de ce mélange en suspension dans un solvant anhydre à l'aide d'une quantité d'acide sulfurique concentré comprise entre environ 0,5 et 1 fois l'équivalence molaire du mélange de leucines, on sépare le précipité qui sera ou non soumis une ou plusieurs fois au même traitement, selon le degré de pureté désiré et on le débarrasse de l'acide sulfurique.

On a constaté en effet que, premièrement, les leucines peuvent former avec l'acide sulfurique des complexes de deux types différents, à savoir un sulfate de formule $2AA.H_2SO_4$ et un sesquisulfate de formule $3AA.2H_2SO_4$, et que, deuxièmement, l'acide sulfurique libre augmente préférentiellement la solubilité du sesquisulfate de leucine, provoquant ainsi l'enrichissement de la phase solide en isoleucine.

Le procédé selon la présente invention permet d'atteindre en un petit nombre d'opérations simples et avec un bon rendement une isoleucine d'un haut degré de pureté à partir d'un mélange encore très chargé en leucine. Le présent procédé s'inscrit donc en complément de procédés industriels d'extraction d'acides aminés à partir de matières végétales ou animales diverses au cours desquels on parvient à isoler des fractions contenant avant tout de l'isoleucine et de la leucine.

Pour mettre en oeuvre le présent procédé, on peut tirer de ce genre de fractions un mélange sec contenant pour la plus grande partie de l'isoleucine et de la leucine.

Il est recommandé de prendre un mélange de départ dans lequel l'isoleucine et la leucine sont dans un rapport pondéral d'au moins 30:70. Avec un rapport inférieur, l'enrichissement du précipité est trop faible, même si le rendement en isoleucine est excellent. Au-dessus de ce rapport, il est possible d'obtenir un enrichissement notable en conservant un rendement appréciable en utilisant une quantité idoine d'acide sulfurique. Par rendement en isoleucine, on entend dans le présent exposé la quantité d'isoleucine finalement recueillie par rapport à la quantité d'isoleucine présente dans le mélange de départ.

Le solvant anhydre recommandé est une cétone aliphatique, notamment la méthyl-éthylcétone. L'acétone donne également de bons résultats, par exemple, mais la méthyl-éthylcétone est préférable pour des raisons techniques en relation avec son point d'ébullition et son point d'inflammation. Dans le même ordre d'idées, on préfère travailler à température ambiante, à savoir aux environs de 20°C. Des températures supérieures à 30°C par exemple impliquent des difficultés techniques et un renchérissement inutiles alors que des températures inférieures à la température ambiante n'apportent aucune amélioration sur le plan de la relation entre la pureté et le rendement.

On peut mettre le mélange de leucines en suspension dans le solvant à raison de environ 5 à 15% en poids de la suspension. En deçà de cette fourchette approximative qui ne représente rien d'autre qu'un compromis raisonnable, la concentration devient trop faible pour que la réalisation industrielle du procédé reste intéressante, même si le rendement en isoleucine et sa pureté en sont d'autant meilleurs. Au delà de cette fourchette, rendement et pureté deviennent insuffisants.

En rapport précisément avec la relation existant entre le rendement et la pureté, la quantité d'acide sulfurique utilisée joue un rôle déterminant. Celle-ci doit être comprise entre environ 0,5 et 1 fois l'équivalence molaire du mélange de leucines. Avec une quantité supérieure, on obtient une dissolution totale des leucines sans qu'il se forme aucun précipité. Avec une quantité inférieure, l'acide sulfurique ne permet pas une dissolution complète des leucines et l'effet d'enrichissement du précipité obtenu est insignifiant, bien que le rendement soit bon. Entre ces limites, la quantité d'acide joue un rôle primordial. Dans les valeurs élevées, le rendement en cristaux est faible mais la teneur en isoleucine est élevée. Dans les valeurs faibles, par contre, les rendements s'améliorent, mais au détriment de la teneur en isoleucine. Dans la pratique, l'opération de purification selon la présente invention pourra être répétée un certain nombre de fois jusqu'à l'obtention du degré de pureté désiré. Pour ce faire, on peut séparer le précipité, le débarrasser ou non de l'acide sulfurique et le réintroduire dans une étape successive analogue de précipitation. La quantité d'acide sulfurique à utiliser dans chaque étape successive est donc de préférence choisie avec grand soin de manière à optimiser le processus et notamment à réduire au minimum le nombre d'étapes nécessaires pour atteindre le degré de pureté voulu sans tomber au-dessous d'un rendement minimum. On peut recommander de choisir cette quantité idoine dans la fourchette comprise entre 60 et 75% en poids du mélange de leucines impliqué dans chaque étape.

C'est ainsi qu'à partir de mélanges de départ contenant l'isoleucine et la leucine dans un rapport pondéral de 50:50 à 90:10 par exemple, on peut obtenir une isoleucine d'une pureté de 98% en 2 à 5 étapes avec un rendement en isoleucine de 15 à 45%.

On peut noter ici que l'opération consistant à débarrasser le filtrat de l'acide sulfurique ne doit présenter aucune difficulté pour l'homme du métier. On peut la réaliser par exemple par neutralisation à l'hydroxyde de barium après élimination du solvant.

Suivant leur teneur respective en isoleucine et en leucine, les eaux-mères issues de chaque étape pourront être soit recyclées à une étape précédente du même procédé soit introduites dans un processus d'extraction de la leucine, par exemple par la voie des chlorhydrates.

La présente invention est illustrée par les exemples suivants dans lesquels les pourcentages et rapports sont donnés en poids.

### Exemple 1

Par le procédé décrit dans le brevet allemand No 24 17 375 on obtient un mélange d'isoleucine et de leucine dans un rapport de 4:1. On met 20 g de ce mélange en suspension dans 400 ml de méthyl-éthylcétone. On ajoute 14,8 g de $H_2SO_4$ à 97% et l'on agite vigoureusement pendant 3 h à 20°C. On filtre la nouvelle phase solide formée qui pèse 14 g. On la dissout dans 200 ml d'eau. On neutralise avec de l'hydroxyde de barium et l'on sépare le sulfate de barium formé. On concentre et sèche le filtrat. On obtient 9,25 g d'une isoleucine contenant encore 5,8% de leucine.

On met ce nouveau mélange d'acides aminés en suspension dans 200 ml de méthyl-éthylcétone. On ajoute 6,4 g d'acide sulfurique à 97% et l'on agite vigoureusement pendant 3 h à 20°C. On filtre les cristaux formés qui pèsent 9,5 g. On les dissout dans 200 ml d'eau et on les neutralise avec du $Ba(OH)_2$. Après filtration du $BaSO_4$ et concentration du filtrat, on obtient 6,1 g de L-isoleucine présentant une pureté de 98%. Le rendement est de 38,1%.

### Exemple 2

On met 100 g d'un mélange contenant 50% de leucine et 50% d'isoleucine dans 2000 ml de méthyléthylcétone. On ajoute 70 g de $H_2SO_4$ à 97% et l'on agite vigoureusement pendant 3 h à 25°C. On filtre les cristaux formés qui représentent un poids de 40,5 g.

On les met un suspension dans 550 ml de méthyl-éthylcétone. On ajoute à la suspension 5,5 g de $H_2SO_4$ à 97%. On agite 3 h à 25°C et on filtre. On obtient 31,5 g de cristaux.

On les met en suspension dans 500 ml de méthyl-éthylcétone contenant 5 g d'$H_2SO_4$ à 97%. On agite 3 h à 25°C et l'on filtre. On obtient 20,8 g de cristaux.

On les met en suspension dans 250 ml de méthyl-éthylcétone contenant 2,5 g de $H_2SO_4$. On agite durant 3 h à 25°C, on filtre et l'on obtient 15,8 g de cristaux.

On les met en suspension dans 250 ml de méthyl-éthylcétone contenant 2,5 g de $H_2SO_4$ à 97%. On agite pendant 3 h à 25°C et l'on filtre. On recueille 12,5 g de cristaux de sesquisulfate d'isoleucine qui, débarrassés de l'acide sulfurique à l'aide de baryte, donnent 8,25 g de l-isoleucine présentant une pureté de 98,2%. Le rendement est de 16,5%.

## Revendications

1. Procédé de purification de l'isoleucine à partir d'un mélange de leucine et d'isoleucine, caractérisé par le fait que l'on précipite l'isoleucine de ce mélange en suspension dans un solvant anhydre à l'aide d'une quantité d'acide sulfurique concentré comprise entre environ 0,5 et 1 fois l'équivalence molaire du mélange de leucines, on sépare le précipité qui sera ou non soumis une ou plusieurs fois au même traitement, selon le degré de pureté désiré et on le débarrasse de l'acide sulfurique.

2. Procédé selon la revendication 1, caractérisé par le fait que le solvant anhydre est une cétone aliphatique, notamment la méthyl-éthylcétone.

3. Procédé selon la revendication 1, caractérisé par le fait que l'on met le mélange en suspension dans le solvant à raison de environ 5 à 15% en poids de la suspension.

4. Procédé selon la revendication 1, caractérisé par le fait que l'on ajoute l'acide sulfurique à raison de 60 à 75% en poids du mélange de leucines.

5. Procédé selon la revendication 1, caractérisé par le fait que dans le mélange de départ l'isoleucine et la leucine sont dans un rapport pondéral d'au moins 40:60.

6. Procédé selon la revendication 1, caractérisé par le fait que l'on débarrasse le précipité de l'acide sulfurique avant de répéter le traitement.

## Patentansprüche

1. Verfahren zur Trennung und Reinigung von Isoleucin aus einem Gemisch von Leucin und Isoleucin, dadurch gekennzeichnet, dass man Isoleucin aus diesem Gemisch in einem wasserfreien Lösungsmittel mittels einer etwa 0,5 bis 1 Mal das Moläquivalent des Gemisches entsprechenden Mench konzentrierter Salzsäure ausfällt und abtrennt, wobei der Niederschlag gegebenenfalls je nach dem ersuchten Reinheitsgrad nochmals oder mehrere Male der gleichen Behandlung unterworfen wird, und der Niederschlag von der Salzsäure befreit.

2. Verfahren nach Anspruch 1, dadurch gekennzeichnet, dass das wasserfreie Lösungsmittel ein aliphatisches Keton, insbesondere Methyläthylketon ist.

3. Verfahren nach Anspruch 1, dadurch gekennzeichnet, dass das Gemisch in einer Menge von etwa 5 bis 15 Gewichtsprozente der Suspension im Lösungsmittel suspendiert wird.

4. Verfahren nach Anspruch 1, dadurch gekennzeichnet, dass die Salzsäure in einer Menge von 60 bis 75 Gewichtsprozente des genannten Gemisches verwendet wird.

5. Verfahren nach Anspruch 1, dadurch gekennzeichnet, dass Leucin und Isoleucin in einem Gewichtsverhältnis von mindestens 40:60 im Ausgangsgemisch enthalten sind.

6. Verfahren nach Anspruch 1, dadurch gekennzeichnet, dass der Niederschlag von der Salzsäure befreit wird, bevor die Behandlung wiederholt wird.

## Claims

1. A process for the purification of isoleucine from a mixture of leucine and isoleucine, characterised in that the isoleucine is precipitated in an anhydrous solvent using a quantity of sulphuric acid of from about 0.5 to 1 time the molar equivalence of the mixture of leucines, the precipitate is separated and optionally subjected once or more times to the same treatment according to the degree of purity to be obtained and the precipitate is freed from sulphuric acid.

2. A process as claimed in Claim 1, characterised in that the anhydrous solvent is an aliphatic ketone, particularly methyl ethyl ketone.

3. A process as claimed in Claim 1, characterised in that the mixture is suspended in the solvent in a quantity of from about 5 to 15% by weight, based on the suspension.

4. A process as claimed in Claim 1, characterised in that the sulphuric acid is added in a quantity of from 60 to 75% by weight, based on the mixture of leucines.

5. A process as claimed in Claim 1, characterised in that the isoleucine and leucine are present in the starting mixture in a ratio by weight of at least 40:60.

6. A process as claimed in Claim 1, characterised in that the precipitate is freed from sulphuric acid before the treatment is repeated.